(19) Europäisches Patentamt / European Patent Office / Office européen des brevets

(11) **EP 1 882 745 A1**

(12) **DEMANDE DE BREVET EUROPEEN**

(43) Date de publication:
**30.01.2008 Bulletin 2008/05**

(51) Int Cl.:
*C12Q 1/00* (2006.01)        *G01N 33/487* (2006.01)

(21) Numéro de dépôt: **06117822.4**

(22) Date de dépôt: **25.07.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(71) Demandeur: **The Swatch Group Research and Development Ltd.**
**2074 Marin (CH)**

(72) Inventeurs:
• **Schuhmann, Wolfgang**
  **44799 Bochum (DE)**
• **Borgmann, Sabine**
  **Fayetteville, AR 72701 (US)**

(74) Mandataire: **Ravenel, Thierry Gérard Louis et al**
**I C B,**
**Ingénieurs Conseils en Brevets SA,**
**7, rue des Sors**
**2074 Marin (CH)**

(54) **Système électrochimique de dosage d'un composé biologique par une enzyme**

(57)     Le système comprend un appareil de mesure électronique destiné à recevoir un capteur électrochimique comportant un substrat supportant les collecteurs de courant permettant de relier les électrodes de mesure et de référence à l'appareil de mesure. L'électrode de mesure est revêtue d'un réactif comprenant au moins l'enzyme spécifique du composé biologique à analyser dans un fluide corporel. L'appareil de mesure permet d'imposer au moins deux températures différentes pour permettant de séparer le signal du composé à analyser de ceux d'autres composés biologiques interférant avec le signal.

Application au dosage du glucose dans le sang avec la glucose déhydrogénase comme enzyme, sans interférence avec le maltose.

Fig. 12

EP 1 882 745 A1

**Description**

Domaine technique

La présente invention concerne un système électrochimique permettant de déterminer le taux d'un composé biologique déterminé dans un échantillon de fluide corporel au moyen d'une enzyme spécifique dudit composé biologique, mais capable d'interférer avec d'autres composés biologiques. L'invention sera plus particulièrement illustrée par le dosage ampérométrique du glucose dans le sang au moyen de la glucose déhydrogénase.

Arrière plan technologique

Un contrôle précis du taux de glucose dans le sang est une précaution vitale pour un grand nombre de patients atteints du diabète, lesdits patients pouvant en outre être astreints en même temps à d'autres traitements pour d'autres troubles de la santé.

Pour connaître ce taux de glucose, qui détermine la quantité d'insuline devant être administrée et faciliter la vie courante de ces malades, il existe déjà de nombreux appareils miniaturisés qui reposent généralement sur une mesure électrochimique par ampérométrique, en présence d'une enzyme spécifique du glucose, l'échantillon de sang étant déposé sur un capteur jetable.

L'enzyme actuellement la plus utilisée est la glucose oxydase (GOD) en raison de sa grande spécificité pour le glucose, à l'exclusion d'autres oligosaccharides, et d'être peu sensible aux variations de température en ce qui concerne la hauteur du signal. La glucose oxydase a toutefois l'inconvénient d'être très sensible à la présence d'oxygène, dont la teneur peut être variable, ce qui fausse la mesure précise du taux de glucose. Pour atténuer, voire éliminer, cet inconvénient il a été proposé d'utiliser un médiateur qui va accélérer le transfert des électrons et rendre négligeable l'influence de l'oxygène. Parmi les médiateurs les plus utilisés, on peut citer le ferrocène et ses dérivés, ainsi que les complexes d'osmium, tels que ceux décrits dans le brevet US 5 393 903.

On peut également utiliser comme enzyme la glucose déshydrogénase (GDH) qui a l'avantage d'être insensible à la présence d'oxygène. La glucose déshydrogénase a toutefois l'inconvénient d'être moins spécifique du glucose et d'interférer avec d'autres saccharides oligosaccharides, oligopolysaccharides tel que le maltose, ce qui conduit à une surestimation du taux de glucose et donc à une administration inappropriée d'insuline. Cela constitue un inconvénient sérieux pour les patients diabétiques qui doivent en outre, par exemple, subir une dialyse péritonéale ambulatoire continue (CAPD) en absorbant de l'icodextrine qui est hydrolysé par l'α-amylase en oligosaccharides tels que le maltose, le maltotriose ou le maltotétrose.

Résumé de l'invention

La présente invention vise donc à procurer un système électrochimique de dosage fiable d'un composé biologique déterminé dans un fluide corporel au moyen d'une enzyme spécifique dudit composé selon un protocole qui permet de discriminer le signal du composé biologique à doser du signal d'autres composés biologiques. Cette discrimination repose fondamentalement sur une sensibilité du composé biologique à doser et des composés biologiques interférants en fonction de la température, ce qui conduit à des cinétiques différentes permettant de séparer les signaux de chaque composé biologique.

L'invention sera plus particulièrement illustrée par un système de dosage du taux de glucose dans un échantillon de sang par des mesures électrochimiques au moyen de la glucose déshydrogénase.

A cet effet le système comprend un appareil de mesure électronique destiné à recevoir un capteur électrochimique et permettant d'imposer au moins deux températures déterminées à l'électrode de mesure de façon à pouvoir discriminer le signal propre au composé biologique à doser du signal d'un autre composé biologique. Le capteur est formé de façon connue par un substrat supportant des collecteurs de courant permettant de relier l'appareil de mesure à au moins une électrode de mesure et une électrode de référence du capteur.

Le réactif déposé sur l'électrode de mesure comprend au moins l'enzyme, mais peut également incorporer un cofacteur de l'enzyme et un médiateur, tel qu'un dérivé du ferrocène ou un complexe d'osmium, facilitant le transfert des électrons entre l'enzyme et l'électrode de mesure.

Selon un premier mode de réalisation les moyens pour porter l'électrode de mesure à au moins deux températures différentes sont constitués par un serpentin chauffant, noyé dans le substrat du capteur et disposé sous l'électrode de mesure tout en étant isolé électriquement de celle-ci. Le serpentin est connecté à l'appareil de mesure qui va envoyer, par exemple, un courant pulsé qui, en fonction d'une calibration préalable, permet d'imposer aux moins deux températures déterminées permettant, par un traitement mathématique des signaux reçus auxdites température de discriminer le signal propre du composé biochimique à doser de celui d'un autre composé biochimique.

Selon un deuxième mode de réalisation le serpentin est intégré à l'appareil de mesure, de telle sorte qu'en introduisant le capteur, la zone de mesure vienne se positionner au-dessus dudit serpentin.

Pour obtenir au moins deux températures différentes au niveau de l'électrode de mesure, l'homme de métier peut concevoir, sans sortir du cadre de la présente invention, un autre dispositif permettant de porter ladite électrode de mesure à deux températures différentes, par exemple au moyen d'un confinement thermostaté de ladite électrode.

Brève description des dessins

D'autres caractéristiques et avantages de la présente invention apparaîtront dans la description suivante, donnée à titre illustratif et non limitatif pour le dosage du glucose dans le sang en utilisant comme enzyme la glucose deshydrogénase, en référence aux dessins annexés dans lesquels :

- La figure 1 est une représentation schématique du matériel de laboratoire utilisé dans le cadre de la présente invention;
- la figure 2 est une représentation simplifiée des réactions au niveau de l'électrode de mesure;
- La figure 3 est un diagramme de calibration du capteur en température;
- les figures 4 et 5 représentent les réponses du capteur à différentes températures en fonction, respectivement des concentrations en glucose ou en maltose;
- les figures 6 et 7 représentent les réponses du capteur à différentes températures en fonction de différentes concentrations en glucose, respectivement sans maltose et avec maltose;
- les figures 8 et 9 représentent en fonction de la température la réponse du capteur pour le glucose et le maltose, respectivement en ce qui concerne l'intensité maximum apparente et la constante de Michaelis apparente;
- la figure 10 est un graphique montrant la corrélation entre les mesures effectuées et la théorie, pour le dosage du glucose sans maltose et en présence de maltose;
- la figure 11 représente un dispositif portable selon l'invention permettant de mesurer un taux de glucose;
- la figure 12 est une représentation agrandie d'un capteur selon un premier mode de réalisation;
- la figure 13 est une représentation agrandie d'un capteur selon un deuxième mode de réalisation, et
- la figure 14 est une représentation agrandie d'une partie de l'appareil de mesure, selon encore un autre mode de réalisation.

Description détaillée d'un mode de réalisation préféré de l'invention

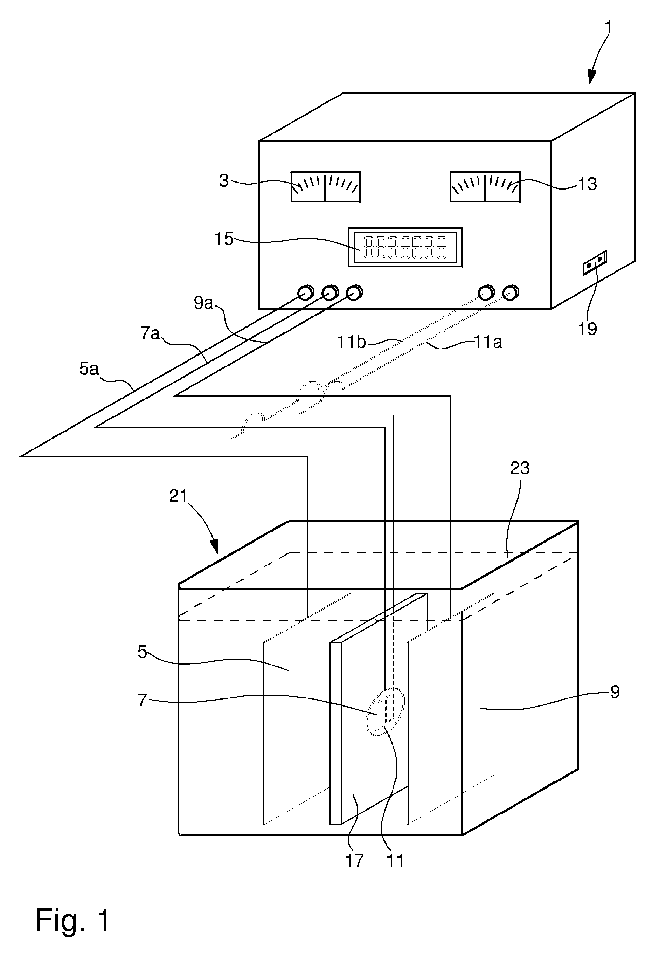

La figure 1 représente schématiquement le matériel de laboratoire qui a été utilisé pour effectuer les mesures et essais rapportés plus loin. Le système comporte un boîtier de commande électronique 1 ayant un potentiostat 3 relié à l'électrode de référence 5 en Ag/AgCl/3M KCl, à l'électrode de mesure 7 et à la contre-électrode 9 toutes deux en platine, respectivement par des connecteurs 5a, 7a et 9a. Le boîtier de commande 1 comporte un deuxième potentiostat 13 dont les câbles de connexion 11a, 11b permettent d'imposer un courant pulsé ou non à un serpentin de chauffe 11 noyé dans un substrat en céramique 17 supportant l'électrode de mesure 7, constituée par un disque de platine de 2 mm de diamètre formé par screen-printing à la surface dudit substrat 17.

La conception du dispositif de chauffe 11 permet de faire varier la température à la surface de l'électrode de mesure 7 sans influencer sensiblement la température du milieu, de préférence sans influencer la température de la masse de la goutte de l'échantillon à tester disposée sur les électrodes. La masse reste donc, de préférence, à température ambiante. Le chauffage du lieu où se déroule les réactions électrochimiques entre l'échantillon et les électrodes sera donc de préférence localisé et rapide.

Le potentiostat 13 en liaison avec une commande associée permet d'imposer plusieurs températures différentes typiquement deux à huit températures, en fonction des caractéristiques d'un courant pulsé comme cela sera expliquée plus loin.

L'appareil comporte en outre un affichage de visualisation 15 de la mesure effectuée et/ou des moyens de connexion 19 pour un appareil électronique (non représenté) permettant, par exemple, d'afficher sous forme graphique le résultat de la mesure en cours, comparativement à des mesures antérieures.

Le système comporte également une cuve 21, représentée à plus grande échelle et contenant un électrolyte 23, apte à recevoir des échantillons de mesure ou de test. Dans toutes les mesures effectuées, l'électrolyte a la composition suivante : 20 mM HEPES, 20 mM KCl et 1 mM $CaCl_2$.

A noter que selon une variante, la cuve 21 peut être omise et dans cas l'échantillon est formé par une goutte directement placée sur les électrodes de mesures comme cela est décrit ci-après.

Dans cet exemple l'électrode de mesure 7 est recouverte d'un réactif comprenant PQQ-sGDH (glucose déshydrogénase avec la pyrroloquinoline quinone comme cofacteur) et un complexe d'osmium agissant comme médiateur pour faciliter le transfert des électrons entre l'enzyme et l'électrode.

Le réactif est de préférence déposé sous forme d'un hydrogel à électrodéposition anodique (EDP), selon une technique connue. Tout autre manière déposer peut également convenir par exemple le dépôt peut être réalisé par un simple pipetage.

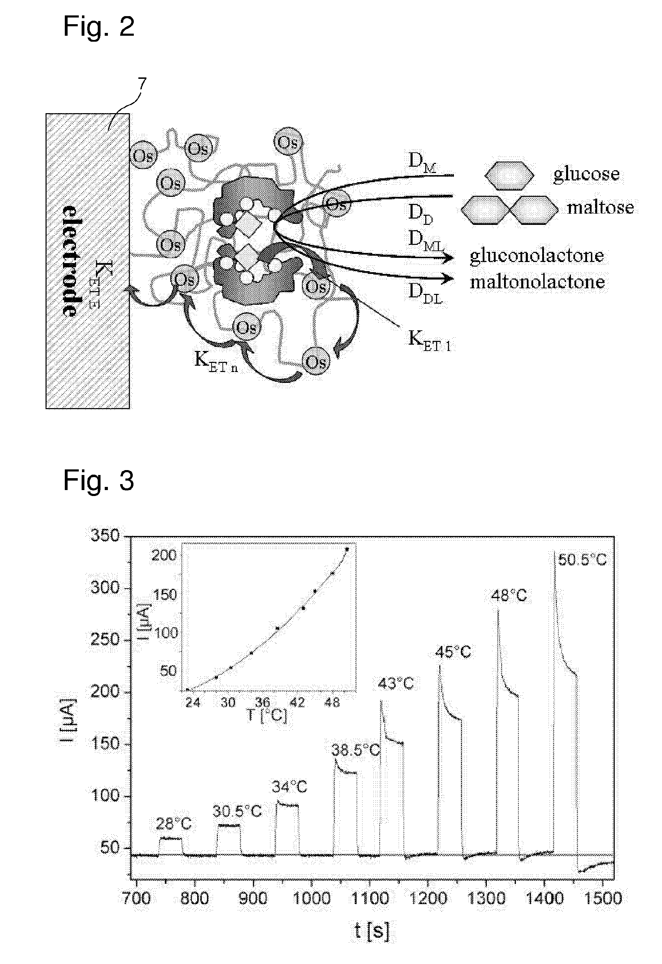

La figure 2 est une représentation simplifiée des réactions à la surface de l'électrode de mesure lorsque le milieu à analyser contient à la fois du glucose et du maltose, qui est un dimère du glucose. Dans ce schéma, $D_M$ représente le coefficient de diffusion du glucose, $D_D$ celui du maltose, $D_{ML}$ celui de la gluconolactone, $D_{DL}$ celui de la maltolactone. $K_{ET\ 1}$ est le coefficient de transfert d'électrons entre la pyrroloquinoline quinone (PQQ) dans le site actif de l'enzyme et le complexe d'osmium de la structure polymère, $K_{ET\ n}$ celui par sauts d'électrons dans le polymère d'osmium et $K_{ET\ E}$ représente l'oxydation électrochimique de $Os^{++}$ en $Os^{+++}$ à la surface de l'électrode de mesure 7.

D'autres enzymes que le glucose déshydrogénase avec d'autres cofacteurs et d'autre médiateurs peuvent bien entendu être envisagés par l'homme de métier pour obtenir le courant représentatifs des réactions chimiques recherchées dont une est notamment illustrée à la figure 2. Typiquement on pourra choisir des médiateurs parmi les composés d'osmium, de ruthénium ou de ferrocènes.

La figure 3 correspond à un diagramme de calibration en température, c'est à dire un diagramme qui permet de connaître la température à la surface de l'électrode de mesure du capteur en fonction d'un courant déterminé appliqué pendant une durée connue. Pour cela on utilise la méthode connue de calibration potentiométrique en appliquant au serpentin chauffant une tension déterminée en mode pulsé au moyen d'un convertisseur DC/AC ou analogue.

Les mesures électrochimiques sont effectuées par ampérométrie en imposant entre les électrodes une tension comprise entre 0 et 750mv selon les caractéristiques de construction du capteur de préférence 300mv.

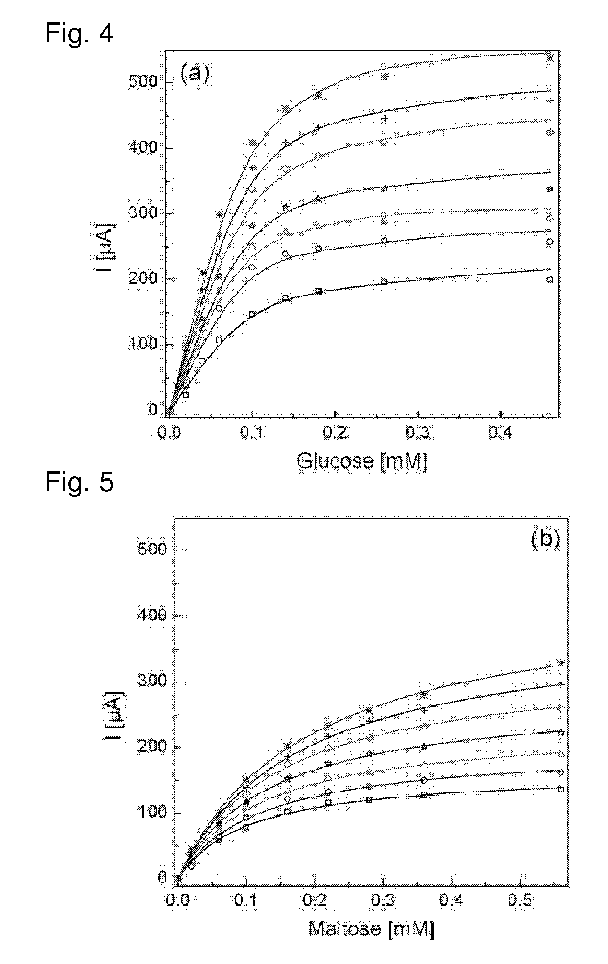

L'exemple de la figure 3 représente le résultat de ces mesures lorsqu'on applique 8 impulsions de 40 secondes chacune déterminant des températures croissantes mesurée à la surface de l'électrode de mesure, tout en enregistrant ampérométriquement en continu le courant du capteur plongé dans un milieu tampon contenant 0,15 mM de glucose. Comme on peut le voir, cela permet de faire varier la température de 28°C à 50,5°C. Comme représenté dans le diagramme inséré, de corréler une intensité appliquée avec une température donnée. Par la suite, on se limitera à une température inférieure à 50°C, car on a observé qu'au delà de cette valeur une baisse d'activité des cofacteurs PQQ. En pratique, dans les essais suivants, on utilise un courant pulsé à 7 impulsions de 30 secondes chacune permettant d'imposer 7 températures différentes représentées sur les graphiques des figures 4 à 7 par les symboles entre parenthèse : 23°C (□) correspondant à la température ambiante, 28°C (0), 30°C (Δ), 34°C (étoile), 38°C (0) 43°C (+), 48°C (*).

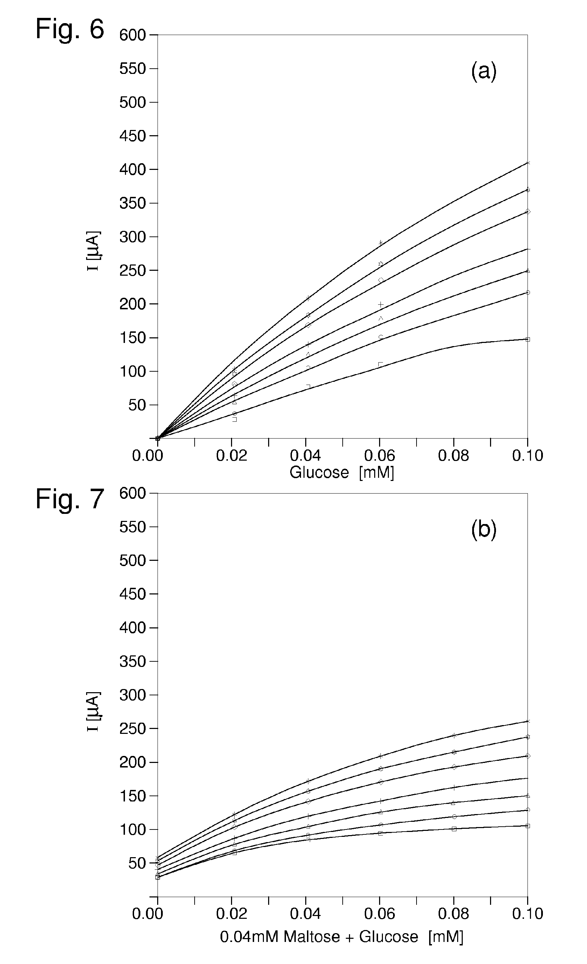

Les figures 4 et 5 représentent graphiquement, respectivement pour le glucose et pour le maltose, des faisceaux de courbes montrant la réponse du capteur pour des concentrations croissantes du composé à analyser aux différentes températures sus-indiquées. Comme on peut le voir, à une même concentration, le courant de réponse du capteur est significativement plus élevé pour le glucose que pour le maltose.

En se référant maintenant aux figures 6 et 7, on a reproduit l'expérience précédente avec des concentrations croissantes de glucose seul (figure 6), puis en présence de 0,04 mM de maltose (figure 7). Comme on peut le voir, la réponse du capteur est moins élevée en présence de maltose, ce qui peut être expliqué par une inhibition compétitive de l'oxydation du glucose en raison du blocage du site actif de l'enzyme par le maltose.

Grâce à ce protocole de chauffage automatisé et à des variations de température parfaitement localisées dans seulement une étroite zone à la surface du capteur, ce qui ne modifie pratiquement pas la température de l'électrolyte, on a pu obtenir un grand nombre de mesures pour différentes concentrations de composés à analyser à différentes températures.

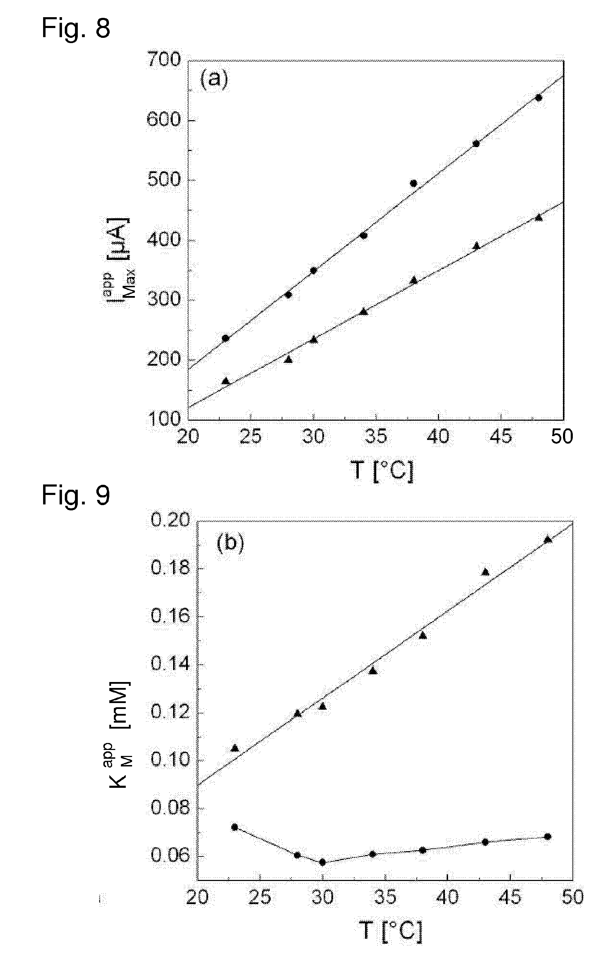

Comme représenté aux figures 8 et 9, on a maintenant évalué les performances du capteur au moyen du courant maximum apparent, $I_{Max}^{app}$ (figure 8) et de la constante apparente de Michaelis, $K_M^{app}$ (figure 9), à la fois pour le glucose (•) et pour le maltose (▲). On peut observer sur la figure 8 une augmentation quasi-linéaire de $I_{Max}^{app}$ pour les deux composés, mesurée en fonction de l'augmentation de température, le signal du glucose étant toutefois un peu plus élevé.

Par contre la figure 9 montre clairement au niveau de $K_M^{app}$ un comportement du glucose et du maltose significativement différent en fonction de l'augmentation de la température. Comme on peut le voir, la valeur du $K_M^{app}$ du glucose n'est pratiquement pas affectée par l'augmentation de la température. Au contraire la valeur du $K_M^{app}$ du maltose montre très nettement une augmentation quasi-linéaire en fonction de la température, ce qui permet de différencier le signal du glucose de celui du maltose.

Compte-tenu des différences de variation du $K_M^{app}$ du glucose et de celui du maltose en fonction de la température, c'est-à-dire en fonction de leurs cinétiques différentes, il est possible de discriminer les signaux et de les séparer par un traitement mathématique par régression linéaire multiple en utilisant comme valeur d'entrée l'intensité du courant à différentes températures et la concentration en glucose comme valeur de sortie.

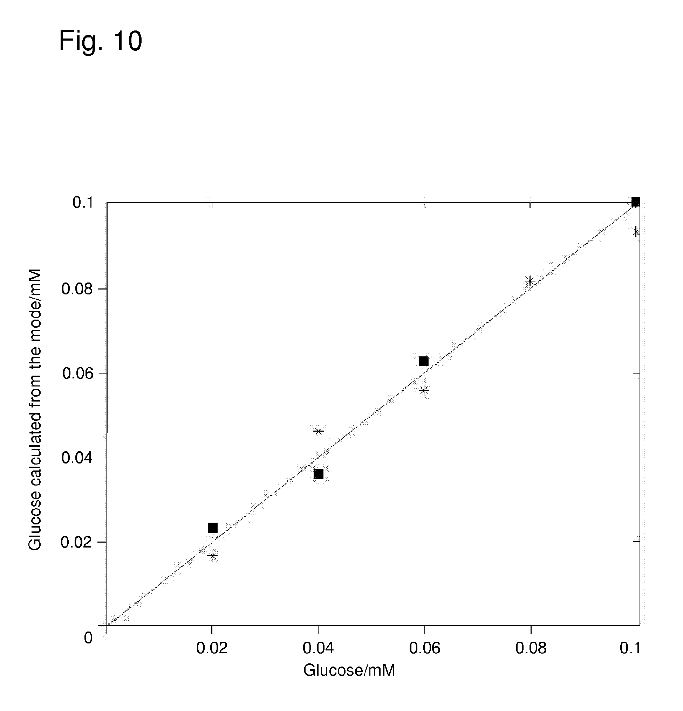

L'expérience a été réalisée sur les résultats obtenus avec un mélange glucose/maltose ayant des concentrations en glucose de 0; 0,02; 0,04; 0,06; 0,08 et 0,1 mM et une concentration en maltose respectivement de 0 mM (■) et de 0,04 mM (*). Le graphique de la figure 10 représente en abscisses le taux de glucose mesuré et en ordonnées le taux glucose calculé. Comme on peut le voir il existe une bonne précision dans la détermination du taux de glucose, qu'on soit ou non en présence de maltose. La bonne performance du model proposé selon l'invention est confirmée d'une part par la valeur élevée du coefficient de détermination R² = 0,968, d'autre part par les faibles valeurs de l'erreur quadratique moyenne RMSE = 0,0018 mM et de l'erreur relative moyenne MRE = 8,8%.

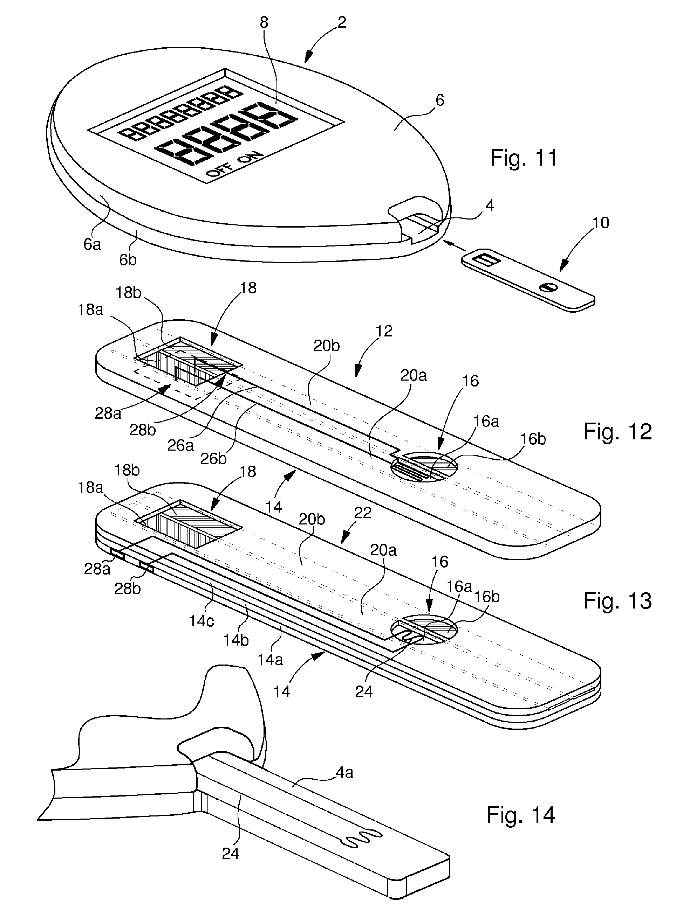

En se référant maintenant aux figures 11 à 14, on a représenté, selon divers modes de réalisation, un appareil portable qui a toutes les fonctionnalités du système décrit à la figure 1, mais dont la miniaturisation permet au patient diabétique de l'avoir avec lui dans tous ses déplacements.

Le dispositif comprend un appareil de mesure 2 destiné à recevoir un capteur électrochimique 10 dans une fente 4 pratiquée dans son boîtier 6, formé de deux coques 6a, 6b. Le boîtier 6 comporte un écran d'affichage 8 permettant de visualiser le résultat d'une mesure. Le boîtier contient une source d'énergie ou est connecté à celle-ci et un circuit électronique (non représentés) permettant à la fois d'imposer une tension déterminée entre les électrodes du capteur 10 et d'alimenter un circuit de chauffage de l'électrode de mesure, comme on le comprendra mieux en référence à la figure 12 qui représente à plus grande échelle un capteur 12 selon un premier mode de réalisation de l'invention.

Dans ce premier mode de réalisation le capteur 12 est réalisé à partir d'un substrat 14 par exemple en céramique frittée multicouches, ou matériaux équivalents, et comporte de façon connue une fenêtre de mesure 16, laissant apparentes l'électrode de mesure 16a et l'électrode de référence 16b, et une fenêtre de connexion 18, laissant apparentes les zones de connexion 18a, 18b. Les électrodes 16a, 16b et les zones de connexion 18a, 18b sont reliées par des pistes conductrices 20a, 20b noyées dans la céramique. Par exemple l'électrode de mesure est revêtue d'un réactif ayant la composition précédemment indiquée, à savoir un réactif contenant au moins PQQ-sGDH et un complexe d'osmium, appliqué sur ladite électrode par électrodéposition anodique d'un hydrogel (EDP) ou analogue.

Le capteur comporte en outre un serpentin chauffant en platine 24 disposé sous l'électrode de mesure 16a en étant noyé dans la céramique. Le serpentin 24 est relié par des conducteurs 26a, 26b à des zones de contact 28a, 28b qui, dans cet exemple sont situés sous les zones de connexion 18a, 18b. Bien évidemment le circuit de chauffage est totalement isolé électriquement du circuit d'alimentation des électrodes. Le capteur 12 qui vient d'être décrit ne comporte que deux électrodes, mais il est bien évident qu'il pourrait également comporter une contre-électrode.

La figure 13 correspond à un deuxième mode de réalisation qui diffère essentiellement du précédent en ce que le substrat 14 est en plastique, Dans l'exemple représenté le substrat 14 est obtenu par l'empilement de trois substrats 14a, 14b, 14c en forme de languettes réalisées en PET qui supportent largement une température ne dépassant pas 50°C. Le substrat inférieur 14a supporte le serpentin chauffant 24 et les conducteurs 26a, 26b allant jusqu'aux zones de contact 28a, 28b, qui dans cet exemple sont ménagées sur un bord du capteur. La face supérieure du substrat intermédiaire 14b supporte les électrodes 16a, 16b, les pistes conductrices 20a, 20b et les zones de connexion 18a, 18b, qui se trouvent ainsi parfaitement isolées électriquement du serpentin de chauffe 24. Le substrat supérieur comporte les fenêtres de mesure 16 et de connexion 18.

Dans les deux modes de réalisation qui viennent d'être décrits, le serpentin de chauffe 24 est intégré dans le capteur lui-même. Selon une variante représentée à la figure 14, le serpentin 14 est séparé du capteur en étant disposé dans un plateau 4a prolongeant la fente 4 de l'appareil de mesure 2, de telle sorte que l'électrode de mesure 16a vienne se positionner sous ledit serpentin 24 lorsque le capteur est engagé dans la fente 4. Dans ces conditions, le capteur peut avoir la forme tout à fait traditionnelle désigné par la référence 10 dans la figure 11, lorsque la détermination du taux de glucose est effectué avec la glucose oxydase.

Sans sortir du cadre de la présente invention, l'homme de métier peut effectuer certaines modifications aux exemples qui viennent d'être décrits, par exemple en amincissant la portion isolante du substrat situé entre l'électrode de mesure et le serpentin de chauffe, ou en utilisant un autre dispositif permettant d'imposer au moins deux températures différentes à l'électrode de mesure.

## Revendications

1. Système de dosage d'un composé biologique déterminé dans un échantillon de fluide corporel par des mesures électrochimiques au moyen d'une enzyme spécifique dudit composé biologique mais susceptible d'interférer avec d'autres composés biologiques, **caractérisé en ce qu'**il comprend :

   - un capteur électrochimique comportant un substrat supportant des collecteurs de courant permettant de relier un appareil de mesure à au moins une électrode de mesure et une électrode de référence, ladite électrode de mesure étant recouverte d'un réactif comprenant au moins l'enzyme spécifique;
   - des moyens, permettant de porter l'électrode de mesure à au moins deux températures déterminées;
   - un appareil de mesure destiné à recevoir le capteur relié à une source d'énergie électrique et comportant un circuit électronique pour commander lesdits moyen permettant de porter l'électrode de mesure à au moins deux températures différentes et pour imposer entre les électrodes une intensité variable ou non, ou une tension variable ou non, et un dispositif d'exploitation des signaux fourni par ledit capteur agencé pour discriminer le signal propre du composé biologique déterminé des signaux résultant d'intéférences avec d'autres composés biologiques.

2. Système de dosage selon la revendication 1, **caractérisé en ce que** les moyens pour porter l'électrode de mesure à au moins deux températures déterminées sont constitués par un serpentin chauffant noyé dans l'épaisseur du capteur et s'étendant sous l'électrode de mesure.

3. Système de dosage selon la revendication 1, **caractérisé en ce que** les moyens de chauffage sont constitués par un serpentin chauffant intégré dans l'appareil de mesure et positionné sous l'électrode de mesure lorsque le capteur est introduit dans ledit appareil de mesure.

4. Système de dosage selon les revendications 2 ou 3, **caractérisé en ce que** les moyens de chauffage sont alimentés par un courant pulsé ou non permettant de porter l'électrode de mesure à au moins deux températures différentes.

5. Système de dosage selon la revendication 1, **caractérisé en ce que** le substrat est réalisée en céramique ou matériaux équivalents.

6. Système de dosage selon la revendication 1, **caractérisé en ce que** le substrat est réalisé en un matériau plastique supportant des températures ne dépassant pas 50°C.

7. Système de dosage selon la revendication 2, **caractérisé en ce que** le substrat est formé par :

   - un substrat inférieur isolant supportant les moyens de chauffage et leurs moyens de connexion à l'appareil de mesure,
   - un substrat intermédiaire isolant supportant à sa surface les électrodes et leurs moyens de connexion à l'appareil de mesure, et
   - un substrat supérieur isolant laissant libres des fenêtres pour les électrodes et les zones de connexion.

8. Système de dosage selon la revendication 1, **caractérisé en ce que** le réactif est déposé sur l'électrode de mesure du capteur par électrodéposition anodique ou par pipetage.

9. Système de dosage selon la revendication 1, **caractérisé en ce que** l'enzyme spécifique entre dans la composition du réactif en étant liée par un cofacteur.

10. Système de dosage selon la revendication 1, **caractérisé en ce que** le réactif comporte en outre un médiateur facilitant le transfert des électrons entre ladite enzyme spécifique et l'électrode de mesure.

11. Système de dosage selon la revendication 1 pour la détermination du taux de glucose dans le sang, **caractérisé en ce que** l'enzyme spécifique est la glucose deshydrogénase.

12. Système de dosage selon la revendication 11, **caractérisé en ce que** la glucose deshydrogénase est présente dans le ractif avec la pyrroloquinoline quinone comme cofacteur (PQQ-sGDH).

13. Système de dosage selon les revendications 10 et 11 **caractérisé en ce que** le médiateur entrant dans la composition du réactif est choisi parmi un dérivé du ferrocene et un complexe d'osmium.

14. Système de dosage selon une quelconque des revendications 11, 12 et 13, **caractérisé en ce qu'**il permet de discriminer un signal propre au glucose d'un signal d'interférence avec d'autres saccharides, oligasachariques ou oligapolysaccarides tels que le maltose.

15. Système de dosage selon la revendication 1, **caractérisé en ce que** les mesures électrochimiques sont effectuées par ampérométrie en imposant entre les électrodes une tension comprise entre 0 et 750mv selon les caractéristiques de construction du capteur de préférence 300mv.

16. Système de dosage selon la revendication 1, **caractérisé en ce que** l'appareil de mesure comporte en outre un écran de visualisation permettant d'afficher le taux de glucose.

17. Système de dosage selon la revendication 16, **caractérisé en ce que** l'appareil de mesure comporte en outre une mémoire permettant d'afficher le résultat de la mesure en cours, comparativement à des mesures antérieures, cet affichage pouvant être effectué par exemple sous forme graphique.

18. Procédé de dosage d'un composé biologique déterminé dans un fluide corporel par des mesures électrochimiques au moyen d'une enzyme spécifique dudit composé biologique, mais susceptible d'interférer avec d'autres composés biologiques, comportant les étapes consistant à

   - déposer un échantillon dudit fluide corporel sur la zone de mesure d'un capteur électrochimique comprenant une électrode de référence et une électrode de mesure revêtu d'un réactif comprenant au moins l'enzyme spécifique;
   - introduire le capteur dans un appareil de mesure relié à une source d'énergie électrique et comportant un circuit électronique permettant, d'une part d'imposer entre les électrodes une intensité variable ou non, ou une tension variable ou non, d'autre part des moyens permettant de porter l'électrode de mesure à au moins deux température différentes
   - soumettre les signaux obtenus, à au moins deux température différentes, à un dispositif d'exploitation permettant de discriminer le signal propre du composé déterminé des signaux résultant d'interférence avec d'autres composés biologiques.

19. Procédé selon la revendication 18, **caractérisé en ce que** les moyens permettant de porter l'électrode de mesure à deux températures différentes sont constitués par intégré dans le capteur ou dans l'appareil de mesure.

20. Procédé selon la revendication 18, **caractérisé en ce que** le dispositif d'exploitation comporte un programme de traitement des signaux par régression linéaire multiple.

21. Capteur électrochimique pour la détermination d'un composé biologique déterminé dans le sang au moyen d'une enzyme spécifique dudit composé biologique comportant au moins une électrode de référence et une électrode de mesure revêtue d'un réactif comportant au moins ladite enzyme spécifique, **caractérisé en ce que** ledit comporte en outre un serpentin chauffant disposé sous l'électrode de mesure et isolé électriquement de celle-ci, ledit serpentin permettant d'imposer à l'électrode de mesure, dans la zone de mesure, au moins deux températures différentes permettant de discriminer le signal propre du composé biologique déterminé des signaux d'interférence avec d'autres composés biologiques.

Fig. 1

# Fig. 2

# Fig. 3

Fig. 4

Fig. 5

Fig. 6

(a)

Fig. 7

(b)

Fig. 8

Fig. 9

# Fig. 10

Fig. 11

Fig. 12

Fig. 13

Fig. 14

EP 1 882 745 A1

# RAPPORT DE RECHERCHE EUROPEENNE

**Office européen des brevets**

Numéro de la demande

EP 06 11 7822

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | JP 06 174679 A (SUMITOMO METAL IND) 24 juin 1994 (1994-06-24) * abrégé; figures 2,5 * | 21 | INV. C12Q1/00 G01N33/487 |
| A | | 1-20 | |
| X | JP 05 256812 A (TOTO LTD) 8 octobre 1993 (1993-10-08) * abrégé; figure 2 * | 21 | |
| A | | 1-21 | |
| X | WO 99/46585 A (USF FILTRATION & SEPARATIONS [US]; HODGES ALASTAIR MCINDOE [AU]; BECK) 16 septembre 1999 (1999-09-16) * page 6, ligne 1 - page 7, ligne 13 * | 21 | |
| A | | 1-20 | |
| X | US 2002/053523 A1 (LIAMOS CHARLES T [US] ET AL) 9 mai 2002 (2002-05-09) * alinéa [0210] - alinéa [0212] * | 21 | |
| A | | 1-20 | DOMAINES TECHNIQUES RECHERCHES (IPC) |
| A | JP 2000 019146 A (MATSUSHITA ELECTRIC IND CO LTD) 21 janvier 2000 (2000-01-21) * abrégé; figure 1A * | 1-21 | G01N C12Q |

Le présent rapport a été établi pour toutes les revendications

| Lieu de la recherche | Date d'achèvement de la recherche | Examinateur |
|---|---|---|
| Munich | 18 décembre 2006 | Komenda, Peter |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

15

**ANNEXE AU RAPPORT DE RECHERCHE EUROPEENNE**
**RELATIF A LA DEMANDE DE BREVET EUROPEEN NO.**

EP 06 11 7822

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-12-2006

| Document brevet cité au rapport de recherche | | | Date de publication | Membre(s) de la famille de brevet(s) | | Date de publication |
|---|---|---|---|---|---|---|
| JP 6174679 | A | | 24-06-1994 | AUCUN | | |
| JP 5256812 | A | | 08-10-1993 | AUCUN | | |
| WO 9946585 | A | | 16-09-1999 | CA | 2322757 A1 | 16-09-1999 |
| | | | | EP | 1064539 A1 | 03-01-2001 |
| | | | | JP | 2002506969 T | 05-03-2002 |
| | | | | TW | 240071 B | 21-09-2005 |
| US 2002053523 | A1 | | 09-05-2002 | AT | 295538 T | 15-05-2005 |
| | | | | AU | 776764 B2 | 23-09-2004 |
| | | | | AU | 1234701 A | 14-05-2001 |
| | | | | CA | 2358993 A1 | 10-05-2001 |
| | | | | CN | 1322299 A | 14-11-2001 |
| | | | | DE | 60020076 D1 | 16-06-2005 |
| | | | | DE | 60020076 T2 | 16-03-2006 |
| | | | | EP | 1145000 A1 | 17-10-2001 |
| | | | | JP | 2003513279 T | 08-04-2003 |
| | | | | JP | 2006091022 A | 06-04-2006 |
| | | | | WO | 0133216 A1 | 10-05-2001 |
| | | | | US | 2002084196 A1 | 04-07-2002 |
| | | | | US | 6616819 B1 | 09-09-2003 |
| JP 2000019146 | A | | 21-01-2000 | AUCUN | | |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5393903 A